(19) **Europäisches Patentamt / European Patent Office / Office européen des brevets**

(11) **EP 4 738 377 A1**

(12) **EUROPEAN PATENT APPLICATION**

(43) Date of publication:
06.05.2026 Bulletin 2026/19

(21) Application number: 25210011.0

(22) Date of filing: 21.10.2025

(51) International Patent Classification (IPC):
$G16H\ 20/30^{(2018.01)}$   $A61B\ 5/00^{(2006.01)}$
$G16H\ 50/20^{(2018.01)}$   $A61B\ 5/024^{(2006.01)}$
$A61B\ 5/08^{(2006.01)}$   $A61B\ 5/11^{(2006.01)}$
$G16H\ 50/30^{(2018.01)}$

(52) Cooperative Patent Classification (CPC):
**G16H 50/20; A61B 5/0205; A61B 5/7264;
G16H 20/30;** A61B 5/02438; A61B 5/0816;
A61B 5/1112; A61B 5/1118; A61B 5/1124;
G16H 50/30

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: 29.10.2024 IN 202421082908

(71) Applicant: **Tata Consultancy Services Limited
Maharashtra (IN)**

(72) Inventors:
• **MAZUMDER, Oishee
700156 Kolkata, West Bengal (IN)**
• **SINHA, Aniruddha
700091 Kolkata, West Bengal (IN)**

• **MUKHERJEE, Ayan
700156 Kolkata, West Bengal (IN)**
• **CHANDEL, Vivek
201301 Noida, Uttar Pradesh (IN)**
• **BHATTACHARYA, Sakyajit
700156 Kolkata, West Bengal (IN)**
• **AHMED, Nasimuddin
700091 Kolkata, West Bengal (IN)**
• **KHANDELWAL, Sundeep
201301 Noida, Uttar Pradesh (IN)**
• **GHOSE, Avik
700091 Kolkata, West Bengal (IN)**

(74) Representative: **Goddar, Heinz J.
Boehmert & Boehmert
Anwaltspartnerschaft mbB
Pettenkoferstrasse 22
80336 München (DE)**

(54) **PERSONALIZED STRUCTURAL AND FUNCTIONAL CARDIAC DIGITAL TWIN FOR ASSESSMENT OF CARDIOPULMONARY ENDURANCE OF ATHLETE**

(57) A method and system that builds a regression model from a personalized structural and functional Cardiac Digital Twin (CDT) for assessment of cardiopulmonary endurance of an athlete is disclosed. The personalized Cardiac Digital Twin (CDT), which replicates echo like functionality under dynamic conditions integrates subject specific kinematics data real time acquired to run personalized CDT and generate intrinsic metrices to evaluate performance in different phases of exercise or endurance activity. Most of existing works are focused on computing mere metrices for entire activity as whole. However, without judicial combination of these metrices obtained in different phases, no meaningful inference can be drawn on performance evaluation.

FIG. 3C

**Description**

CROSS-REFERENCE TO RELATED APPLICATIONS AND PRIORITY

The present application claims priority to Indian application no. 202421082908, filed on October 29, 2024.

TECHNICAL FIELD

The embodiments herein generally relate to the field of machine learning and predictive analytics and, more particularly, to a method and system for personalized structural and functional Cardiac Digital Twin (CDT) for assessment of cardiopulmonary endurance of an athlete.

BACKGROUND

Health digital twins are essentially digital replicas of human organs, like heart, liver, etc. emulating its functional properties that can be used in for individualized prediction of different treatment outcomes with the goal to virtually select the most promising strategy. Modelling human heart or creating a Cardiac Digital Twin (CDT) of the heart can revolutionize cardiac healthcare in precision medicine and therapy management domain. Such models can also be envisaged for other applications that requires predictive analysis, and high endurance athletic cardiac remodeling is a perfect example where these models can provide groundbreaking insights and discoveries into various parameters effecting the cardiac health and athletic performance.

Utilization of CDT has been primarily used in medical domain and its application in athletic training or stress activities for enhanced predictive analytics is open area for research.

SUMMARY

Embodiments of the present disclosure present technological improvements as solutions to one or more of the above-mentioned technical problems recognized by the inventors in conventional systems.

For example, in one embodiment, a method for personalized structural and functional Cardiac Digital Twin (CDT) for assessment of cardiopulmonary endurance of an athlete is provided. The method includes time synchronizing sensor data acquired during an endurance activity performed by each subject among a plurality of subjects, wherein the sensor data represents a plurality of data types comprising ECG data, accelerometer data providing speed, Gravity data, GPS data and Barometer data acquired from a plurality of sensors worn by each subject, and wherein the plurality of subjects are a mix of a professional athlete, a mid-level athlete and an amateur athlete.

Further, the method includes segmenting each of the plurality of data types into a plurality of segments. A first step comprises segmenting by identification of i) an initial resting or warmup segment, ii) an Intense Activity (IA) segment and iii) a recovery segment post the IA segment based on Heart Rate (HR) variation and associated Metabolic Equivalent Task (MET). A second step comprises segmenting the IA segment into i) an initial ramp-up, ii) a cruise, iii) an occasional dip in speed, and iv) an occasional increase in speed and/or heartrate.

Further, the method includes running a personalized Cardiac Digital Twin (CDT) model, built for each subject, on corresponding segments of each of the plurality data types to extract a plurality of sets of cardiopulmonary dynamics. A plurality of sets of cardiopulmonary features are derived from the plurality of sets of cardiopulmonary dynamics for each subject, and wherein a distribution of the sets of cardiopulmonary features is processed via a feature transformation technique to obtain a transformed cardiopulmonary feature vector for each subject;

Furthermore, the method includes extracting a set of kinematic features from one or more of the of the plurality of data types acquired during the endurance activities. A distribution of the set of kinematic features is processed via a feature transformation technique to obtain a transformed kinematic feature vector for each subject.

Further, the method includes generating an annotated feature matrix comprising a plurality of features vectors representing the plurality of subjects via the transformed kinematic feature vector concatenated with the transformed cardiopulmonary feature vector, wherein each feature vector among a plurality of feature vectors of the annotated feature matrix is annotated with a proficiency score of each of the subject for the endurance activity.

Furthermore, the method includes creating trained data regression models using the annotated feature matrix for predicting the proficiency score for the professional athlete, the mid-level athlete and the amateur athlete.

During inference personalized guidance and training plan for future runs of a test subject is generated based on the predicted proficiency score, a personalized CDT of the subject, and a set of kinematic and cardiopulmonary features extracted for the test subject.

In another aspect, a system for personalized structural and functional Cardiac Digital Twin (CDT) for assessment of cardiopulmonary endurance of an athlete is provided. The system comprises a memory storing instructions; one or more Input/Output (I/O) interfaces; and one or more hardware processors coupled to the memory via the one or more I/O interfaces, wherein the one or more hardware processors are configured by the instructions to time synchronize sensor data acquired during an endurance activity performed by each subject among a plurality of subjects, wherein the sensor data represents a plurality of data types comprising ECG data, accelerometer data providing speed, Gravity data, GPS data and Barometer data acquired from a plurality of sensors worn by each subject, and wherein the plurality of subjects are a mix of a professional athlete, a mid-level athlete and an amateur athlete.

Further, the one or more hardware processor are configured to segment each of the plurality of data types into a plurality of segments. A first step comprises segmenting by identification of i) an initial resting or warmup segment, ii) an Intense Activity (IA) segment and iii) a recovery segment post the IA segment based on Heart Rate (HR) variation and associated Metabolic Equivalent Task (MET). A second step comprises segmenting the IA segment into i) an initial ramp-up, ii) a cruise, iii) an occasional dip in speed, and iv) an occasional increase in speed and/or heartrate.

Further, the one or more hardware processor are configured to run a personalized Cardiac Digital Twin (CDT) model, built for each subject, on corresponding segments of each of the plurality data types to extract a plurality of sets of cardiopulmonary dynamics. A plurality of sets of cardiopulmonary features are derived from the plurality of sets of cardiopulmonary dynamics for each subject, and wherein a distribution of the sets of cardiopulmonary features is processed via a feature transformation technique to obtain a transformed cardiopulmonary feature vector for each subject;

Furthermore, the one or more hardware processor are configured to extract a set of kinematic features from one or more of the of the plurality of data types acquired during the endurance activities. A distribution of the set of kinematic features is processed via a feature transformation technique to obtain a transformed kinematic feature vector for each subject.

Further, the one or more hardware processor are configured to generate an annotated feature matrix comprising a plurality of features vectors representing the plurality of subjects via the transformed kinematic feature vector concatenated with the transformed cardiopulmonary feature vector, wherein each feature vector among a plurality of feature vectors of the annotated feature matrix is annotated with a proficiency score of each of the subject for the endurance activity.

Furthermore, the one or more hardware processor are configured to create trained data regression models using the annotated feature matrix for predicting the proficiency score for the professional athlete, the mid-level athlete and the amateur athlete.

During inference personalized guidance and training plan for future runs of a test subject is generated based on the predicted proficiency score, a personalized CDT of the subject, and a set of kinematic and cardiopulmonary features extracted for the test subject.

In yet another aspect, there are provided one or more non-transitory machine-readable information storage mediums comprising one or more instructions, which when executed by one or more hardware processors causes a method for personalized structural and functional Cardiac Digital Twin (CDT) for assessment of cardiopulmonary endurance of an athlete. The method includes time synchronizing sensor data acquired during an endurance activity performed by each subject among a plurality of subjects, wherein the sensor data represents a plurality of data types comprising ECG data, accelerometer data providing speed, Gravity data, GPS data and Barometer data acquired from a plurality of sensors worn by each subject, and wherein the plurality of subjects are a mix of a professional athlete, a mid-level athlete and an amateur athlete.

Further, the method includes segmenting each of the plurality of data types into a plurality of segments. A first step comprises segmenting by identification of i) an initial resting or warmup segment, ii) an Intense Activity (IA) segment and iii) a recovery segment post the IA segment based on Heart Rate (HR) variation and associated Metabolic Equivalent Task (MET). A second step comprises segmenting the IA segment into i) an initial ramp-up, ii) a cruise, iii) an occasional dip in speed, and iv) an occasional increase in speed and/or heartrate.

Further, the method includes running a personalized Cardiac Digital Twin (CDT) model, built for each subject, on corresponding segments of each of the plurality data types to extract a plurality of sets of cardiopulmonary dynamics. A plurality of sets of cardiopulmonary features are derived from the plurality of sets of cardiopulmonary dynamics for each subject, and wherein a distribution of the sets of cardiopulmonary features is processed via a feature transformation technique to obtain a transformed cardiopulmonary feature vector for each subject;

Furthermore, the method includes extracting a set of kinematic features from one or more of the of the plurality of data types acquired during the endurance activities. A distribution of the set of kinematic features is processed via a feature transformation technique to obtain a transformed kinematic feature vector for each subject.

Further, the method includes generating an annotated feature matrix comprising a plurality of features vectors representing the plurality of subjects via the transformed kinematic feature vector concatenated with the transformed cardiopulmonary feature vector, wherein each feature vector among a plurality of feature vectors of the annotated feature matrix is annotated with a proficiency score of each of the subject for the endurance activity.

Furthermore, the method includes creating trained data regression models using the annotated feature matrix for predicting the proficiency score for the professional athlete, the mid-level athlete and the amateur athlete.

During inference personalized guidance and training plan for future runs of a test subject is generated based on the predicted proficiency score, a personalized CDT of the subject, and a set of kinematic and cardiopulmonary features extracted for the test subject.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 is a functional block diagram of a system for building a regression model trained on a personalized structural and functional Cardiac Digital Twin (CDT) for assessment of cardiopulmonary endurance of an athlete, in accordance with some embodiments of the present disclosure.

FIGS. 2A and 2B (collectively referred as FIG. 2) is a flow diagram illustrating a method for regression model trained on the personalized CDT for assessment of cardiopulmonary endurance of an athlete, using the system depicted in FIG. 1, in accordance with some embodiments of the present disclosure.

FIGS. 3A through 3D depict overall system architecture and process flow for the regression model trained on the personalized CDT for assessment of cardiopulmonary endurance of an athlete, in accordance with some embodiments of the present disclosure.

FIGS. 4 through 14E are graphical depiction of relation among parameters associated with fitness state of subjects during performance evaluation for endurance activities, in accordance with some embodiments of the present disclosure

It should be appreciated by those skilled in the art that any block diagrams herein represent conceptual views of illustrative systems and devices embodying the principles of the present subject matter. Similarly, it will be appreciated that any flow charts, flow diagrams, and the like represent various processes which may be substantially represented in computer readable medium and so executed by a computer or processor, whether or not such computer or processor is explicitly shown.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Digital cardiac models, such as a Cardio Vascular (CVR) Model and others have been proposed for physical performance analysis of subjects. Models such as the CVR Model estimate cardio-dynamic parameters (changes in cardiac output, stroke volume, and heart rate), regional blood flow, and muscle oxygen extraction, in response to rest and physical workloads, across a range of ages and aerobic fitness levels, as well as during exposure to heat, dehydration, and altitude. However, evaluating real performance measures for sportspersons or athletes that can truly contribute to enhancing their training regimes requires further granularity analysis across the activity by capturing more significant parameters at various stages of endurance activity during runtime.

For example, the finish time in a marathon competition is known to all the runners. However, in a 2-3 hours (Hrs) duration of the run, the dynamics of the pacing, distribution of the speed at which various distances are covered, onset time of fatigue, oxygen demand are not analyzed in holistic manner. The important parameters those are usually observed using various sports wearables (e.g. Garmin watch with Strava application) are pace, distance, time (duration), heartrate, VO2max etc. It also provides information on the change in such parameters, longitudinally. This of course helps the runners to understand the improvement that happens over weeks of practice, before an upcoming competition. However, none of the currently available sports applications relate those parameters with the cardiovascular functions (hemodynamic and electrophysiology) of an individual, neither they provide mechanistic explanations on the observed parameters or can predict the cardio-pulmonary recovery trend after such endurance exercise. The key asks of the marathon runners are the following:

1. Even if there is not much of a change in total time, has my cardio-pulmonary performance improved? If yes, then I can push towards higher heartrate zone for longer durations.
2. Where do I stand in endurance measure as compared to the champion runners?

3. In a full marathon run, how is my pacing profile and cardio-pulmonary performance different from the champion runners?

Embodiments herein provide a method and system that builds a regression model from a personalized structural and functional Cardiac Digital Twin (CDT) for assessment of cardiopulmonary endurance of an athlete. The personalized Cardiac Digital Twin (CDT), which replicates echo like functionality under dynamic conditions integrates subject specific kinematics data real time acquired to run personalized CDT and generate intrinsic metrices to evaluate performance in different phases of exercise or endurance activity. Most of existing works are focused on computing mere metrices for entire activity as whole. However, without judicial combination of these metrices obtained in different phases, no meaningful inference can be drawn on performance evaluation.

The method disclosed acquires sensor data capturing endurance activity of amateur, mid-level and professional runners, segments it into a plurality of segments based on intensity of the activity and based on variations observed within the high intensity segment. The personalized CDT is then run over these segments to obtain cardiopulmonary dynamics, further processed to derive cardiopulmonary features. Similarly kinematic features are obtained from the senor data. Thus, the personalized CDT digital enables establishing a link between increasing kinematics and matched cardiac response for the subject being monitored.

An annotated feature matrix comprising a plurality of feature vectors is generated, wherein each feature vector is concatenation of a kinematic feature vector and a cardiopulmonary feature vector for each subject generated from distribution of cardiopulmonary features or metrices and kinematic features or metrices for each individual. The annotated feature matrix has endurance performance score annotation and is then used to obtain a trained regression model for proficiency score prediction.

During inference stage, a personalized guidance and training plan is generated derived from difference of the kinematic and cardiopulmonary metrices for the test subject from the professional athlete and the mid-level athlete depending upon the predicted proficiency score of the test subject to identify a plurality of measures to be focused upon for improvement with reference the a mid-level athlete later progressing towards the professional athlete or an amateur athlete progressing towards mid-level.

The segmentation of acquired sensor data and cardiopulmonary dynamics performed by the method disclosed for generating feature vectors for training the regression model for proficiency score prediction, allows identification of key phases on which cardiac energetics and other cardiopulmonary metrices are computed. Regression model incorporates features computed from these phases and not the complete exercise tenure (as whole). Thus, the method provides guidance is more phase specific manner, based on autodetection of phases and generating cardiopulmonary energetics, which involves computational and data modeling linkage to predict/recommend modification in phases of exercises.

The following are some of the objectives of the method and system disclosed herein.

1. For athletes of all abilities (amateur and elite / pro runners) to train, compete, recover and manage their overall cardiovascular health with personalized insights.
2. To integrate data with 3D models to truly visualize digital twin hearts and gain better insights.
3. Provide personalized heart insights to help runners train, compete, and recover optimally. Potential insights include:

Endurance: Heartrate at a given speed and their relationship with Cardio-pulmonary functions during running
Recovery: How quickly the heartrate returns to normal
Demonstrate how training for a marathon changes a heart - An MRI, echocardiogram, and training data provide the information needed to compare a runner's heart at the beginning and end of a training cycle as well as compare the hearts of different marathoners.

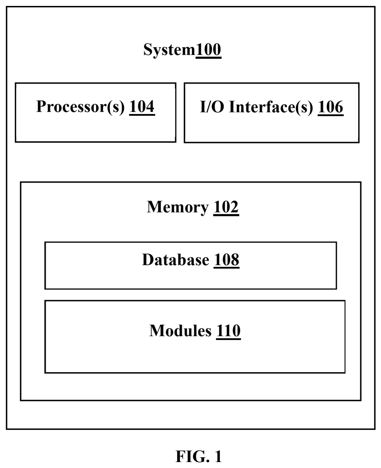

Referring now to the drawings, and more particularly to FIGS. 1 through 14E, where similar reference characters denote corresponding features consistently throughout the figures, there are shown preferred embodiments, and these embodiments are described in the context of the following exemplary system and/or method.

FIG. 1 is a functional block diagram of a system 100 for building a regression model trained on a personalized structural and functional Cardiac Digital Twin (CDT) for assessment of cardiopulmonary endurance of an athlete, in accordance with some embodiments of the present disclosure. In an embodiment, the system 100 includes a processor(s) 104, communication interface device(s), alternatively referred as input/output (I/O) interface(s) 106, and one or more data storage devices or a memory 102 operatively coupled to the processor(s) 104. The system 100 with one or more hardware processors is configured to execute functions of one or more functional blocks of the system 100.

Referring to the components of system 100, in an embodiment, the processor(s) 104, can be one or more hardware processors 104. In an embodiment, the one or more hardware processors 104 can be implemented as one or more microprocessors, microcomputers, microcontrollers, digital signal processors, central processing units, state machines, logic circuitries, and/or any devices that manipulate signals based on operational instructions. Among other capabilities, the one or more hardware processors 104 are configured to fetch and execute computer-readable instructions stored in the memory 102. In an embodiment, the system 100 can be implemented in a variety of computing systems including laptop computers, notebooks, hand-held devices such as mobile phones, workstations, mainframe computers, servers, and the like.

The I/O interface(s) 106 can include a variety of software and hardware interfaces, for example, a web interface, a graphical user interface and the like and can facilitate multiple communications within a wide variety of networks N/W and protocol types, including wired networks, for example, LAN, cable, etc., and wireless networks, such as WLAN, cellular and the like. In an embodiment, the I/O interface (s) 106 can include one or more ports for connecting to a number of external devices or to another server or devices. The I/O interface 106 can source the sensor data captured for real time parameters of each subject user observation for a plurality of internal and external databases. The sourced information can be stored in a database 108 within the memory 102.

The memory 102 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes.

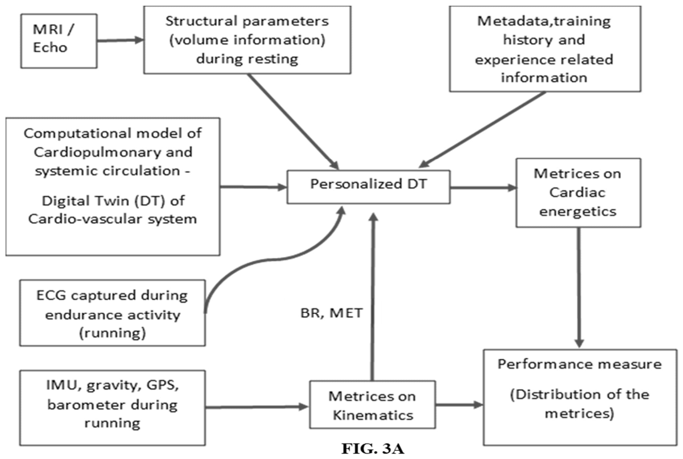

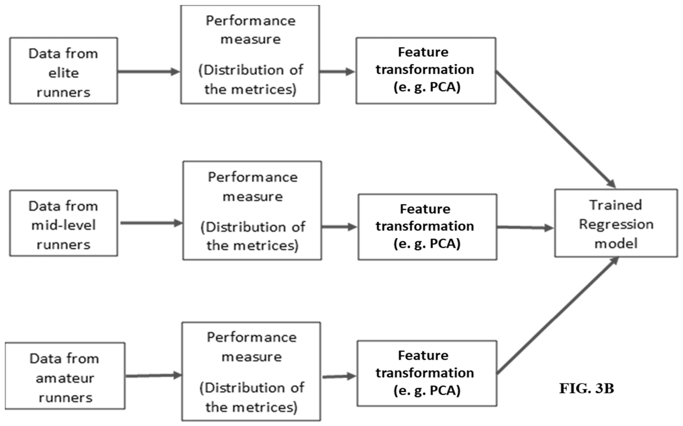

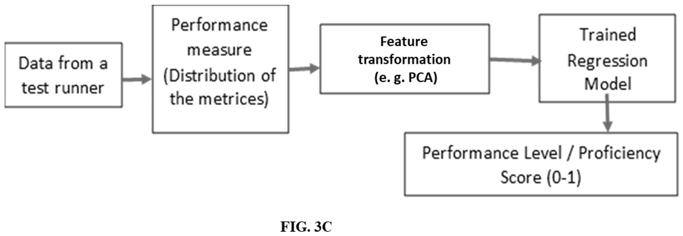

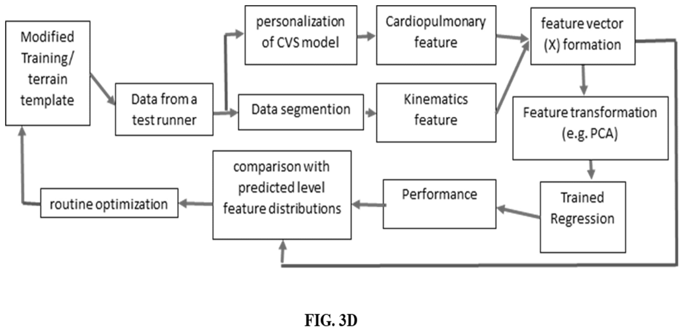

In an embodiment, the memory 102 includes a plurality of modules 110 such as a personalized CDT model (depicted in FIG. 3A), a trained regression model (depicted in FIG. 3B, 3C and 3D) and the like for each individual subject for data is collected for generating training data for a regression model.

The plurality of modules 110 include programs or coded instructions that supplement applications or functions performed by the system 100 for executing different steps involved in the process of generating the trained regression model from the personalized CDT. The plurality of modules 110, amongst other things, can include routines, programs, objects, components, and data structures, which performs particular tasks or implement particular abstract data types. The plurality of modules 110 may also be used as, signal processor(s), node machine(s), logic circuitries, and/or any other device or component that manipulates signals based on operational instructions. Further, the plurality of modules 110 can be used by hardware, by computer-readable instructions executed by the one or more hardware processors 104, or by a combination thereof. The plurality of modules 110 can include various sub-modules (not shown).

Further, the memory 102 may comprise information pertaining to input(s)/output(s) of each step performed by the processor(s) 104 of the system100 and methods of the present disclosure.

Further, the memory 102 includes the database 108. The database (or repository) 108 may include a plurality of abstracted pieces of code for refinement and data that is processed, received, or generated as a result of the execution of the plurality of modules in the module(s) 110. The database 108 also can store the generated feature vectors and annotated feature matrix derived from cardiopulmonary features and kinematic features, also referred as metrices.

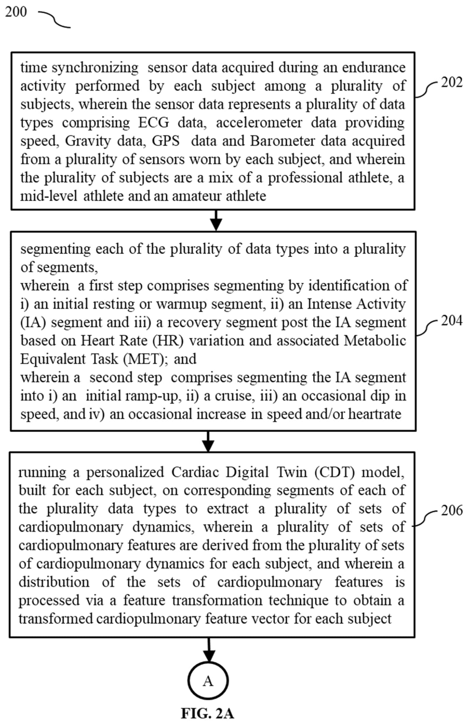

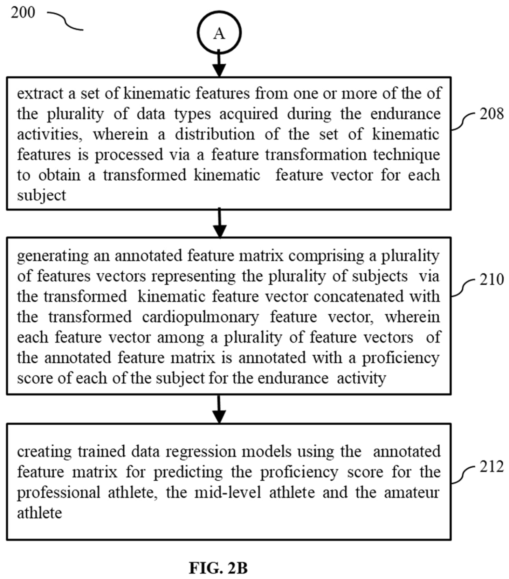

Although the database 108 is shown internal to the system 100, it will be noted that, in alternate embodiments, the database 108 can also be implemented external to the system 100, and communicatively coupled to the system 100. The data contained within such external database may be periodically updated. For example, new data may be added into the database (not shown in FIG. 1) and/or existing data may be modified and/or non-useful data may be deleted from the database. In one example, the data may be stored in an external system, such as a Lightweight Directory Access Protocol (LDAP) directory and a Relational Database Management System (RDBMS). Functions of the components of the system 100 are now explained with reference to steps in flow diagram in FIG. 2 and FIGS. 3A through 14 E.

FIGS. 2A and 2B (collectively referred as FIG. 2) is a flow diagram illustrating a method 200 for regression model trained on the personalized CDT for assessment of cardiopulmonary endurance of an athlete, using the system depicted in FIG. 1, in accordance with some embodiments of the present disclosure.

In an embodiment, the system 100 comprises one or more data storage devices or the memory 102 operatively coupled to the processor(s) 104 and is configured to store instructions for execution of steps of the method 200 by the processor(s) or one or more hardware processors 104. The steps of the method 200 of the present disclosure will now be explained with reference to the components or blocks of the system 100 as depicted in FIG. 1, the steps of flow diagram as depicted in FIG. 2 and system architecture with end to end process flow as depicted in FIGS. 3A, 3B, 3C and 3D. Although process steps, method steps, techniques or the like may be described in a sequential order, such processes, methods, and techniques may be configured to work in alternate orders. In other words, any sequence or order of steps that may be described does not necessarily indicate a requirement that the steps be performed in that order. The steps of processes described herein may be performed in any order practical. Further, some steps may be performed simultaneously.

Referring to the steps of the method 200, at step 202 of the method 200, the one or more hardware processors 104 are configured by the instructions to time synchronize sensor data acquired during an endurance activity performed by each subject among a plurality of subjects. The sensor data represents a plurality of data types comprising electrocardiogram (ECG) data, accelerometer data providing speed, Gravity data, Global Positioning System (GPS) data and Barometer data acquired from a plurality of sensors worn by each subject. The plurality of subjects are a mix of a professional athlete, a mid-level athlete and an amateur athlete. The endurance activity for example can be a sports event, for example, running (marathon), or triathlon that includes multiple types of high intensity sports such as cycling, swimming and running and the like. To get sample data, wearable sensor data (single lead ECG, accelerometer, GPS) is captured over 3 months during the practice sessions.

The sensor data acquisition for all participating subject is performed in accordance with applicant's granted Indian patent applications listed below, and not detailed herein for brevity.

• METHOD AND SYSTEM FOR DIGITAL BIOMARKERS PLATFORM

Granted Patent Number 545836

• ACCELERATION-BASED STEP ACTIVITY DETECTION AND CLASSIFICATION ON MOBILE DEVICES

Granted Patent Number 439754

• CALORIE ESTIMATION patent granted no. 368504

Example data collection for an event such as Marathon, is explained below:
1. Metadata through initial questionnaire:

Table 1

| Particip ant ID | Gend er | Ag e | How many years have you been a runner ? | How many FULL maratho ns have you compet ed in? | Heigh t (feet, inche s) | Basal (restin g) Heart Rate | VO 2 ma x | Best 5 km finish (HH:MM: SS) | Best 10 km finish (HH:MM: SS) | Best 21 km finish (HH:MM: SS) |
| --- | --- | --- | --- | --- | --- | --- | --- | --- | --- | --- |
| | | | | | | | | | | |

Table 2

| What training plan do you follow for marathon preparation? Do you employ any particular running technique or formula, pacing, etc.? Please share any information we may find helpful in calibrating the data you share each week. | Do you employ any specific injury avoidance mea- sures during in- tense training ses- sion? If yes, please describe here. If no, you may skip this question. | To elevate your training and performance experience, what data points (that you aren't measuring today) would you find most helpful? What is your wish list in terms of helping you train to run a marathon? | To help calibrate the data you record and share during our study, it's important for us to understand where your phone is secured. When running and re- cording data with Polar H10 device, where do you attach your phone? |
| --- | --- | --- | --- |

2. Baseline Clinical Tests:
As part of the baseline tests, for each runner Cardiac MRI, Echocardiography, 12-lead Electrocardiogram, Cardiopulmonary Exercise Test and Fat measurements are planned.
3. Longitudinal data using wearable sensors
For every week, sensor data collection is planned for one indoor exercise session and one outdoor running session. A total of approximately 10-12 weeks are planned. In each session, the data is captured for a couple of hours using the following devices.
4.
(i) A chest wearable device Polar H10 for capturing continuous ECG. iPhone paid application is used for the same (e.g. https://play.google.com/store/apps/details?id=com.mattimononen.ecglogger& hl=en_US&gl=US).
(ii) iPhone to capture Accelerometer and GPS. This would use iPhone's inbuilt sensors and there is no need for wrist- watch. Standard iPhone application (e.g. Sensor Logger app (https://apps.apple.com/mr/app/sensor-logger/ id1531582925) downloaded from Apple play store is used for logging the accelerometer and GPS. The accelerometer is used to estimate the intensity of exercise and GPS is used outdoors to estimate the speed-distance profile during running. List of sensor logs to be enabled is given below:

Table 3

| Sensor type | Sampling Frequency |
|---|---|
| Accelerometer | 40 Hz |
| Gravity | 40 Hz |
| GPS | 2 Hz |
| Barometer | 2 Hz |

At step 204 of the method 200, the one or more hardware processors 104 are configured by the instructions to segmenting each of the plurality of data types into a plurality of segments. A first step comprises segmenting by identification of i) an initial resting or warmup segment, ii) an Intense Activity (IA) segment and iii) a recovery segment post the IA segment based on Heart Rate (HR) variation and associated Metabolic Equivalent Task (MET). A second step comprises segmenting the IA segment into i) an initial ramp-up, ii) a cruise, iii) an occasional dip in speed, and iv) an occasional increase in speed and/or heart rate.

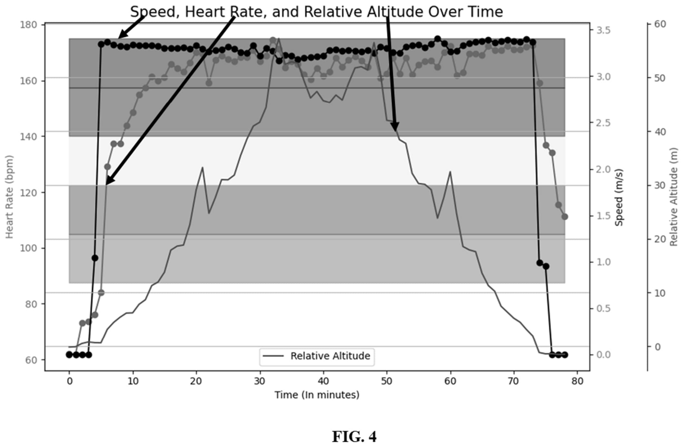

The extraction of segments, interchangeably referred to as phases, which is a two-step process, the system 100 first identifies these phases from HR variation and associated MET. For example, as can be seen in FIG. 4, start phase is expected to have least HR variation and 0 MET level. Similarly, Ramp phase is identified as the phase which has a sudden high +ve gradient in HR and MET.

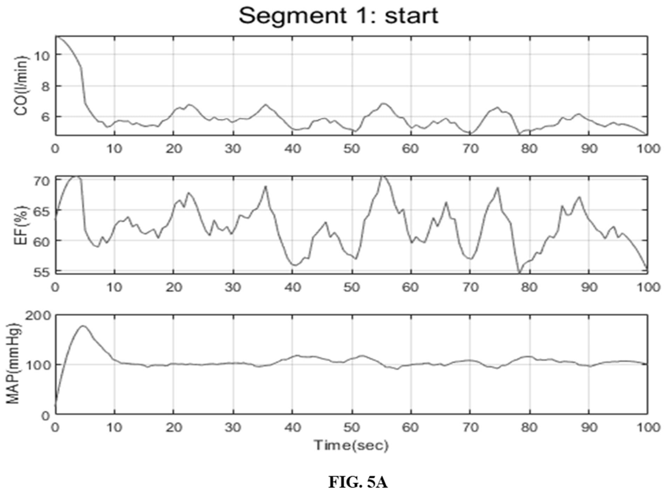

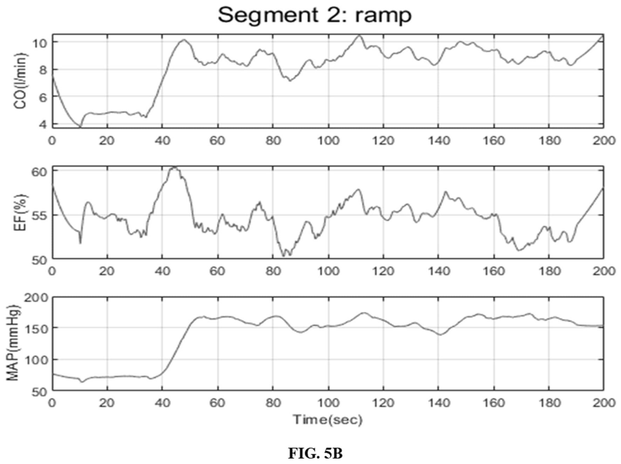

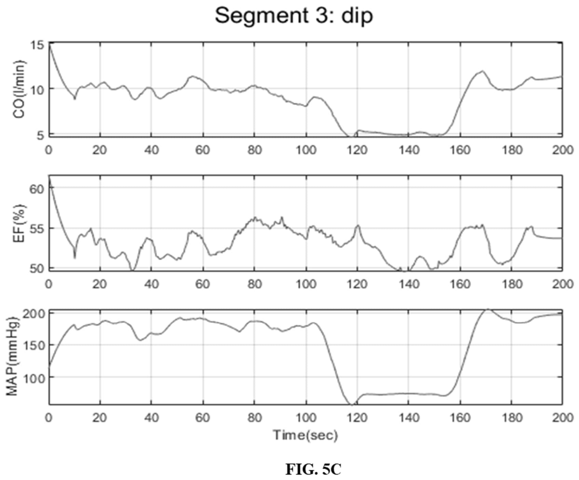

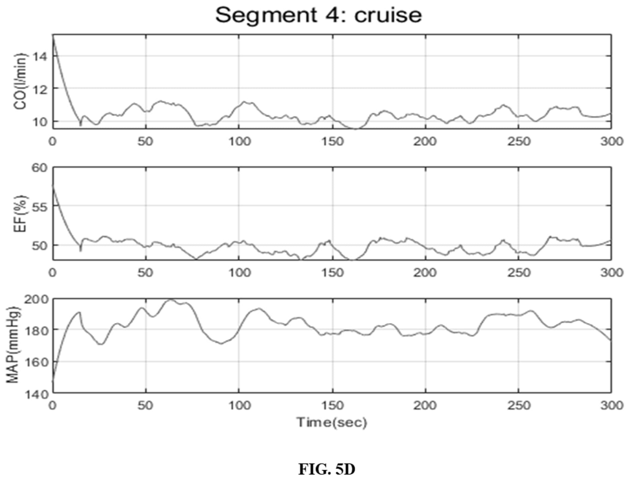

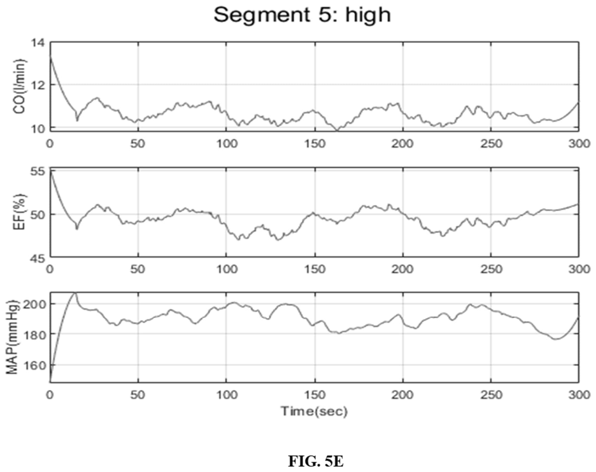

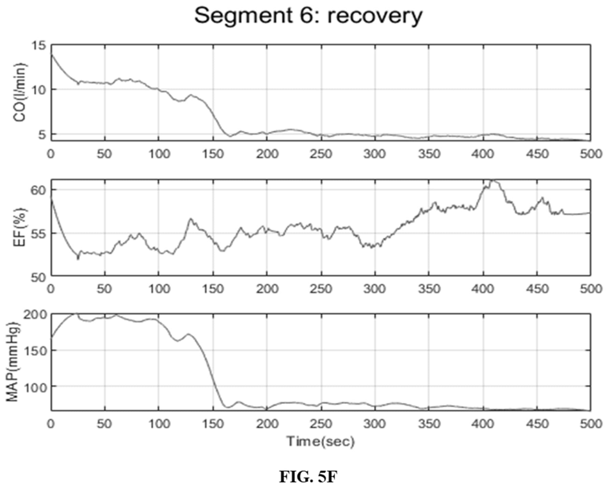

The sample segments corresponding to various phases of endurance activity are depicted in FIGS. 5A through 5E.

At step 206 of the method 200, the one or more hardware processors 104 are configured by the instructions to run the personalized Cardiac Digital Twin (CDT) model, built for each subject, on corresponding segments of each of the plurality data types to extract a plurality of sets of cardiopulmonary dynamics. The CDT model generation and working is in accordance with applicant's Indian patent applications listed here. Method And System For Pressure Autoregulation Based synthesizing Of Photoplethysmogram Signal", Indian Patent Application - 201921029536, AND ESTIMATING CARDIAC PARAMETERS WHEN PERFORMING AN ACTIVITY USING A PERSONALIZED CARDIOVASCULAR HEMODYNAMIC MODEL", Indian Patent Application - 202121010972. Not detailed herein for brevity. The CDT is a computational model replicating cardiac hemodynamics and electrophysiology functioning integrated on the 3d cardiac structure of an individual heart, created from subject specific MRI data. The CDT model is personalized using the baseline clinical parameters as collected in step above along with the metadata (e.g. age, height, weight, body mass index (BMI), body surface area (BSA)) of the individuals. In true sense, the cardiac model is a digital replica of a person's beating heart. This structural-functional cardiac twin now is driven using activity data provided by seasoned/amateur athletes during their training sessions. Along with the kinematics information (cadence, running efficiency, calorie burnt, pacing, etc.) derived from these running sessions, additional insights related to cardiopulmonary functioning during different stages of the run could be extracted. Some examples: like change in cardiac output with pacing, how mean arterial pressure is varying when a runner is transitioning from aerobic to tempo pacing zone, what is the change in perfusion to ventilation ratio, how is the running economy, insights into cardiac contractility and elastance during peak exercise durations and recovery, how is the pulmonary blood flow changing with increase in activity intensity, changes in cardiac workload and probable comparison between subjects or same subject over different training sessions.

A plurality of sets of cardiopulmonary features are derived from the plurality of sets of cardiopulmonary dynamics for each subject. The distribution of the sets of cardiopulmonary features is processed via a feature transformation technique to obtain a transformed cardiopulmonary feature vector for each subject. The set of cardiopulmonary features comprise metabolic equivalent of task (MET), (av) arteriovenous, (per) perfusion, pva: pressure volume area, mep: mean power, VE: ventricular efficiency, Heart rate (HR), Energy ejected (EE), Stroke work (SW), mep: mean power, VE: ventricular efficiency, ESP end systolic pressure, EDV: end diastolic volume, ESPVR: end systolic pressure volume ratio, EDPVR: end diastolic pressure volume ratio, Mean power (Pmean), Cardiac output (CO), Stroke volume (SV), Ejection Fraction (EF), and Mean arterial pressure (MAP).

Thus, once the phases and associated segments are identified, the personalized CDT model is run on the specified segments to generate cardiac flow, volume and pressure dynamics. Left ventricle dynamics part is of interest herein. As each segment is of varying length (few minutes to several minutes), the cardiac dynamics is ever changing with each beat, reflecting the HR variations. For any segment, the cardiac model is run over the complete duration of the segments (each segment comprises of multiple windows of 20,000 samples), but one representative beat is selected to compute energetics and dynamics related metrics. The representative beat is selected from the last 20,000 sample segment window, once the transient response settles and a clean cycle can be obtained.

At step 208 of the method 200, the one or more hardware processors 104 are configured by the instructions to extract a set of kinematic features from one or more of the of the plurality of data types acquired during the endurance activities, wherein a distribution of the set of kinematic features is processed via a feature transformation technique to obtain a transformed kinematic feature vector for each subject. Thus, the kinematics information like total distance, total time, pacing, heartrate, cadence, metabolic equivalent task (MET), calorie burnt etc, are extracted. The breathing rate is extracted from the envelop of the ECG signal. The elevation information along the running track is extracted using GPS and gravity information. The weather information (temp, humidity, rain etc.) during the run is extracted using GPS.

The set of kinematics features comprise Work Intensity (WI), Running VO2 (VO2run), Average running efficiency (REavg), REconomy, Average heart rate (HRavg), Average breathing rate (BRavg), Session time (ST), Calorie (Cal), maximum speed (Smax), Average Cadence (Cadavg), Average MET (METavg), and Total distance (TD).

At step 210 of the method 200, the one or more hardware processors 104 are configured by the instructions to generating an annotated feature matrix comprising a plurality of features vectors representing the plurality of subjects via the transformed kinematic feature vector concatenated with the transformed cardiopulmonary feature vector. Each feature vector among a plurality of feature vectors of the annotated feature matrix is annotated with a proficiency score of each of the subject for the endurance activity. Thus, a pproficiency score [0-1] for each subject for different endurance activity types is annotated for features vectors in the feature matrix.

At step 212 of the method 200, the one or more hardware processors 104 are configured by the instructions to create trained data regression models using the annotated feature matrix for predicting the proficiency score for the professional athlete, the mid-level athlete and the amateur athlete.

During inference personalized guidance and training plan for future runs of a test subject is generated based on the predicted proficiency score, a personalized CDT of the subject, and a set of kinematic and cardiopulmonary features extracted for the test subject. The personalized guidance and training plan generation comprises determining a difference of the kinematic features and cardiopulmonary features (interchangeably also referred to as metrices as they are one among the many performance indicators of the subject) for the test subject from the professional athlete and the mid-level athlete depending upon the predicted proficiency score of the test subject to identify a plurality of measures to be focused upon for improvement with reference the a mid-level athlete later progressing towards the professional athlete or an amateur athlete progressing towards mid-level.

**USE CASE:** Following steps 202 through 208, the following features are derived for sample subjects (for example herein, runners).

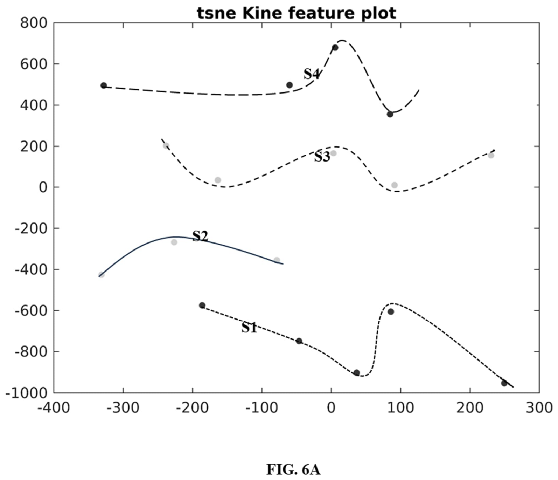

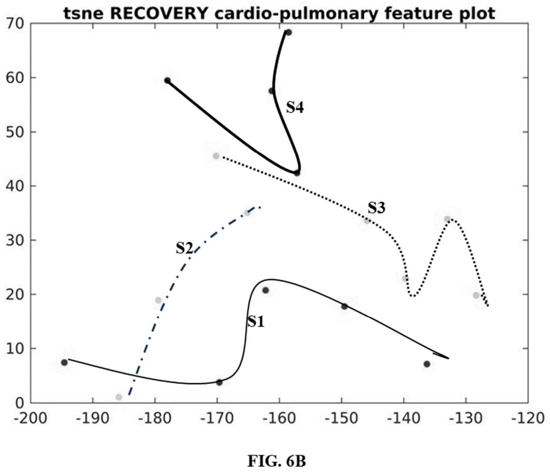

a. Kinematic feature (KF) = [WorkIntensity RunningVO2 avgRE REconomy avg HR avg BR Session Time (min) Calorie (Kcal) max speed(m/sec) avg Cadence avg MET Total Distance (Km)]
b. Cardiopulmonary feature (CPF)= [HR MET av PER PVA mep VE HR EE sw mep ESP EDV espvr edpvr EF CO SV MAP]
c. Please note: A) the complete feature-space is not limited to above-mentioned above feature set. It is rather a representative list. B) The CP features are extracted from the recovery phase; the kinematic features are obtained from the complete run.
d. Two t-SNE (t-distributed Stochastic Neighbor Embedding) plots (typically used for high-dimensional data visualizing through unsupervised non-linear dimensionality reduction) corresponding to the CPF and KF vectors corresponding to subjects of varying levels of expertise in endurance sports (like elite, Intermediate, inexperienced represented by s1, s2, s3, s4, wherein 3,4 are pro runners) are given in FIGS. 6A and 6B respectively. It can be observed that clear non-overlapping clusters can be formed, that affirms the efficacy of the selected features.

1. For the $i^{th}$ runner, the feature vector (Xi) is constructed by stacking the above-mentioned metric/features values.

$$Xi = [KFi\ CPFi]$$

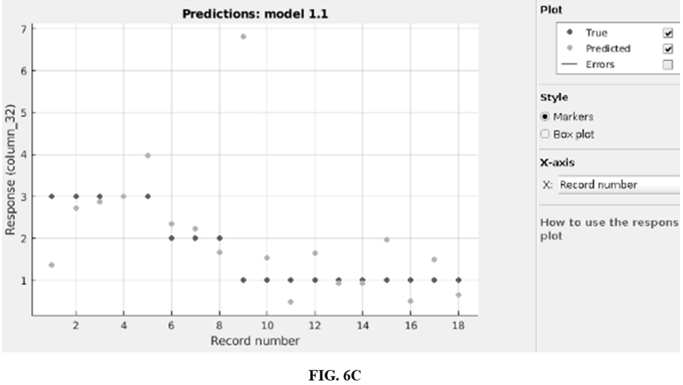

The feature vector would be passed through a typical feature transformation module (PCA or likewise). The corresponding independent variable $Y_i$ (class label) could be a numeric rank of the performance. Now, $X_l$ would be the feature matrix containing the features vectors for all the runners (sample subjects) during their run and $Y_l$ would be the rank vector. Now after appropriate preprocessing of $X_l$, a multi-variate regression model of the form $Y = A \cdot X + b$ will be trained using $\{X_l, Y_l\}$. Here $X$ is the independent variable, here b is the constant and $A$ denote the Regression Coefficient. The output is a normalized score [0-1]. A typical regression process can be Gaussian process regression. The plot of the true (black) and predicted (grey) proficiency levels is given in FIG. 6C.

Following are metrices derived from the analysis of the data. Metrices computed can be broadly classified in two groups:

1. Kinematic metrices derived using sensor data (ECG and accelerometer).

2. After the CDT model has been personalized with athlete specific metadata, structural information and exercise related inputs derived from the sensor data (during running/ exercise), cardio-pulmonary parameters are obtained from the personalized CDT model with running kinematic parameters. The techniques of obtaining the parameters are as described in applicants patent applications filed at Indian Patent office, as listed below, and not explained for brevity:

- METHOD AND SYSTEM FOR PRESSURE AUTOREGULATION BASED SYNTHESIZING OF PHOTOPLETHYSMOGRAM SIGNAL, Application Number 201921029536
- A METHOD AND SYSTEM FOR SOURCE LOCALIZATION OF ATRIAL FIBRILLATION, Application Number 202321080595
- ESTIMATING BLOOD PRESSURE OF A SUBJECT USING AN ECG DRIVEN CARDIOVASCULAR MODEL, Application Number 202221033450

ESTIMATING CARDIAC PARAMETERS WHEN PERFORMING AN ACTIVITY USING A PERSONALIZED CARDIOVASCULAR HEMODYNAMICMODEL, Application Number 202121010972. The endurance parameters for comparing performance of athletes are derived combining kinematics and cardiopulmonary metrices. A comprehensive list of all the computed metrices is provided below:

i. Baseline cardiac structure and functions from MRI, Echo and Cardiac Digital Twin
ii. Kinematics - pace, cadence, MET using mobile phone sensors (using acktrack IP as mentioned above, IN Patent 368504 (filed 03/06/2021) IN Patent 439754 (filed 20/07/2023) IN Patent 545836 (filed 24/07/2024))
iii. **Endurance/Fitness metrics** computed from athlete specific sensor data:
**HR zone:** easy, aerobic, tempo, lactate threshold and anaerobic zone distribution. Individual zone computed w,r,t. age matched max HR (220-age). **VO2 related metrics**

1) **Running VO2 (VO2_running):** $\dot{V}O_2 = 3.5 + (0.2 \times S) + (0.9 \times S \times G)$ [s- speed (m/min), G grade (elevation in %)]
2) **% V02 Max (running intensity)** = 0.8 + 0.1894393 * exp (-0.012778 * time) + 0.2989558 * exp (-0.1932605 * times)
3)

$$\mathbf{VO2} = -4.60 + 0.182258 * velocity + 0.000104 * velocity\verb|^|2$$

4)

$$\mathbf{VO2\ MAX}\ (\text{also known as vdot}) = VO2\ /\ \text{percent max}$$

**5) Exercise intensity (I)= Activity duration (avg HR-RHR)/(MHR-RHR);** Where RHR: resting HR, MHR: Max HR
6) Running economy refers to the energy demand of running at a given submaximal velocity.

$$\mathbf{Reco} = VO2 * 60\ \text{min/hr} * BM\ (kg)^{-1} * \text{Running Speed (km/h)}$$

7) Running efficiency is a measure of the ratio of work done to energy expended.

$$\mathbf{RE} = \text{speed/hr (mph/bpm)}$$

8) Calorie consumed: MET* WEIGHT* ACTIVITY DURATION.

iv. **Ventilation based metrics:** These metrics are derived combining kinematics and cardiac model generated parameters:

**Minute ventilation (VE):** Tidal volume* respiratory rate (RR)
[Tidal volume: 8-10 ml/kg of wt]
RR computed from ECG sensor data (proprietary algo??)
**Ventilation Perfusion ratio: VE/Qp**

Qp is the blood flow in pulmonary circulation, generated by the cardiac model. Arterio venous difference: [It is an indication of how much oxygen is removed from the blood in capillaries as the blood circulates in the body]

$$CA02\text{-}CV02 = VO2\ Running/\ CO$$

CO is cardiac output (Stroke volume*HR) generated using cardiac model, Vo2 Running from endurance metrics

v. Recovery scores (exercise/running vs recovery) - How fast the HR reduces after the running / exercise is over (IDF: 1345948 009)

vi. HRV related features - SDNN, NN20, NN50, LF/HF etc.

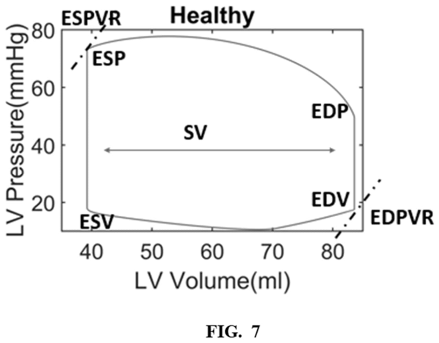

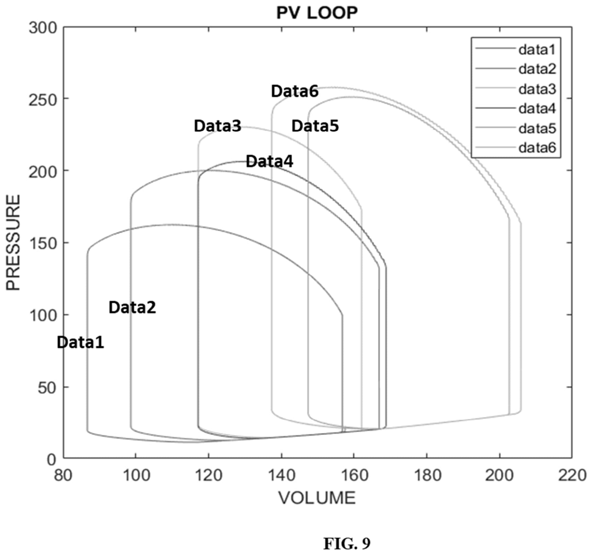

vii. Cardiac model generated parameters - CO, SV (End diastolic volume (EDV)-End Systolic volume(ESV)), Ejection Fraction (EF=SV/EDV*100), MAP (Mean arterial pressure: 1/3 SBP+2/3 DBP), all derived using Left ventricle PV loop dynamics during exercise[1]. The cardiac model generates pressure, volume and flow dynamics in each cardiac cycle using a lumped functional hemodynamic model that replicates cardiac pumping action, considering 4 heart chambers with compliant behaviour, synchronous valve functioning and systemic and pulmonary circulation, guided by cardiac contractility and Central nervous system control. A sample PV loop with areas of interest provided for reference in FIG. 7 and FIG. 9.

List of Features:

1. Work Intensity (WI)
2. Running VO2 (VO2run)
3. Average running efficiency (REavg)
4. Average heart rate (HRavg)
5. Average breathing rate (BRavg)
6. Session time (ST)
7. Calorie (Cal)
8. maximum speed(Smax)
9. Average Cadence(Cadavg)
10. Average MET (METavg)
11. Total distance (TD)
12. Energy ejected (EE)
13. Stroke work (SW)
14. Mean power (Pmean)
15. Ionotropy (Iono)
16. Elasticity (EL)
17. Afterload (AL)
18. Preload (PL)
19. Heart rate (HR)
20. Cardiac output (CO)
21. Stroke volume (SV)
22. Ejection Fraction (EF)
23. Mean arterial pressure (MAP)

A feature vector (X) is constructed by stacking the above-mentioned metric/features values. Thus, a typical feature vector $(X_{i,j})$ would consist of the above feature values as obtained/computed for the $i_{th}$ runner during the $j_{th}$ segment of the run. The corresponding independent variable $Y_{i,j}$(class label) could be a numeric rank of the performance. Now $\mathbf{X_{I,j}}$ would be the feature matrix containing the features vectors for all the runners during the $j_{th}$ segment of the run and $\mathbf{Y_{I,j}}$ would be the rank vector. Now after appropriate preprocessing of $\mathbf{X_{I,j}}$, a multi-variate regression model of the form $\mathbf{Y = A \cdot X + b}$ will be trained using $\{\mathbf{X_{I,j}},\ \mathbf{Y_{I,j}}\}$. Here $\mathbf{X}$ is the independent variable, here b is the constant and A denote the Regression Coefficient.

**RESULTS: for Subjects (S1 to S4)**

Table 4

| Parameter | S2 (F) | S4 (F) | S1 (M) | S3 (M) |
|---|---|---|---|---|
| Age | 29 | 41 | 53 | 29 |
| No of marathons completed | 3-6 marathons | > 10 marathons | 1-2 marathons | 3-6 marathons |
| Active years | More than 12 years | More than 12 years | 3-6 years | More than 12 years |
| Avg running in week in distance | 41-60 miles | > 60 miles | < 20 miles | > 110 miles |
| VO2max | 47.2 ml/kg/min | 56 ml/kg/min | 59.4 ml/kg/min | 80 ml/kg/min |
| Height/Weight | 5'8" / 129 pounds | 5'1" / 98 pounds | 6'0" / 155 pounds | 5'11" / 139 pounds |
| BMI/BSA | BMI-19.6 / BSA-1.68 m2 | BMI-18.5 / BSA-1.38 m2 | BMI-21 / BSA-1.89 m2 | BMI-19.4 / BSA-1.78 m2 |
| LVED Diameter (short axis) in mm | 57.5 mm | 56.1 mm | 53 mm | 46 mm |
| LVEDD index | 34.23 mm/m2 | 40.65 mm/m2 | 28.04 mm/m2 | 25.84 mm/m2 |
| LVES Diameter (short axis) in mm | 39.6 mm | 39.6 mm | 34.37 mm | 31 mm |
| LVED length in mm | 87 mm | 86.8 mm | 86.3 mm | |
| LVES length in mm | 63.74 mm | 74.2 mm | 76.07 mm | |
| Resting Heart rate | 55 | 54 | 61 | 44 |
| LVED Volume | 150.5 ml | 143.04 ml | 126.93 ml | 142 ml |
| LVEDVI = LVEDV/BSA | 89.6 m1 / m2 | 103.7 ml /m2 | 67.16 ml / m2 | 79.78 ml / m2 |
| LVES ml | 52.3 ml | 60.9 ml | 47.13 ml | 51.8 ml |
| LVSV ml | 98.2 ml | 82.12 ml | 79.8 ml | 90.2 ml |
| LVSVI = LVSV/BSA | 58.45 ml / m2 | 59.51 ml /m2 | 42.22 ml / m2 | 50.67 ml / m2 |
| LVEF in % | 65.3% | 57.4% | 61.4 % | 64 % |
| CO (resting) | 5.4 1 | 4.43 1 | 4.87 l | 3.971 |
| Cardiac Index = CO/BSA | 3.2 l/m2 | 3.21 l/m2 | 2.58 l/m2 | 2.23 l/m2 |
| RVED transverse | 43.9 mm | 47.16 mm | 44.67 mm | |
| RVES transverse | 29.61 mm | 37.19 mm | 36.19 mm | |
| Fractional transverse change (FTC) | 0.326 | 0.211 | 0.19 | |
| RVED longitudinal | 105.9 mm | 92.07 mm | 79.14 mm | |
| RVES longitudinal | 85.02 mm | 75.79 mm | 60.22 mm | |
| Fractional longitudinal change (FLC) | 0.197 | 0.177 | 0.239 | |
| T/L = FTC/FLC | 1.655 (Overloaded to constricted) | 1.194 (normal) | 0.795 (towards degeneration) | |
| RVED Volume | 118.3 ml | 106.7 ml | 86.35 ml | |
| RVEDVI = RVEDV / BSA | 70.42 ml / m2 | 77.32 ml / m2 | 45.69 ml / m2 | |
| RVES Volume | 48.48 ml | 60.46 ml | 45.92 ml | |

(continued)

| Parameter | S2 (F) | S4 (F) | S1 (M) | S3 (M) |
|---|---|---|---|---|
| Age | 29 | 41 | 53 | 29 |
| RVSV | 69.82 ml | 46.24 ml | 40.3 ml | |
| RVEF in % | 59.02 % | 43.3 % | 46.8 % | |

Kinematics:

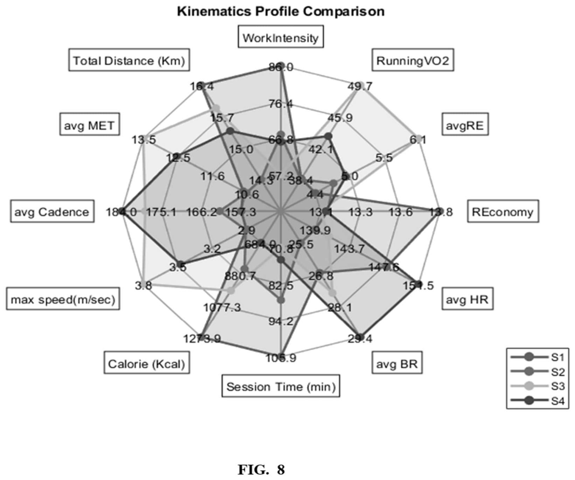

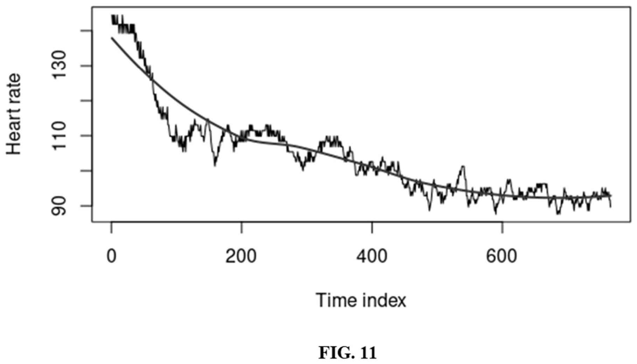

FIG. 2 depicts variations in heartrate, speed and altitude is shown for a sample run. FIG. 8 depicts distribution of heartrate zone is shown for a running session. FIG. 11 depicts recovery profile of the heartrate is shown for a sample run.

FINDINGS:

With reference to FIG. 8, Professional (Pro) runners kinematic metrices (avg pace, cadence, duration, distance, etc.) are much better than recreational runners. Pro runners can sustain higher heart rate zones (lactate threshold and anaerobic zones) yet feel much less exertion compared to recreational runners.

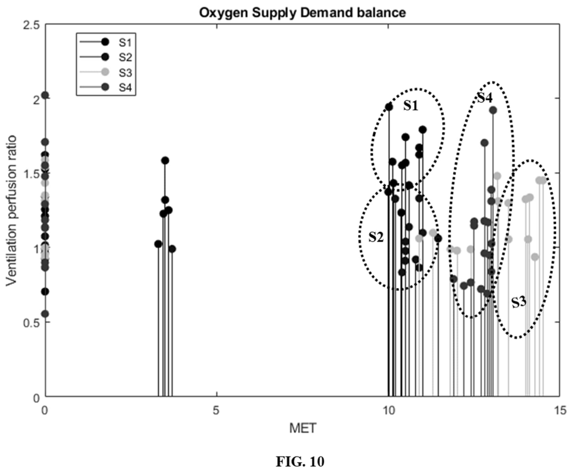

As seen from FIG. 10, ventilation perfusion ratio, which is an indication of oxygen supply demand balance, varies between athletes based on their session kinematics. Compared to recreational runners, Pro runners can achieve higher MET levels with similar Ventilation perfusion ratio. Ventilation perfusion ratio gives information on oxygen intake through lungs (we use the information on breathing rate) and amount of blood flow through pulmonary circulation. Usually this is close to 1 meaning the amount of O2 taken by lungs is just enough for oxygenating the blood flowing through the lungs. Lower values indicate that the breathing is not sufficient and higher values indicate that excess breathing is being done. The ideal ventilation perfusion ratio is 1 or a bit less than 1. Pro runners can achieve higher MET levels with similar Ventilation perfusion ratio. In other words, pro runners achieve higher MET levels while using the same amount of oxygen. MET is the metabolic equivalent task. It reflects the energy expenditure, i.e. how much energy one uses. Higher values indicate that the speed is high. However, MET is a function of speed and BMI, BSA.

Arterio venous oxygen difference is notably higher during running at the higher altitude location, requiring them to inhale greater volume of O2 for similar MET levels. The arteriovenous oxygen difference (AVO2 diff) is the difference in the oxygen content of the blood between the arterial blood and the venous blood. It is an indication of how much oxygen is removed from the blood in capillaries as the blood circulates in the body.

Recovery pattern of pro runners is depicted in FIG. 11. This signifies that an intentional lowering in pace (or rest of few sec) can quickly bring down heart rate and cardiac exertion. These in turn help the runner to maintain their pace for longer duration with less perceived exertion.

Cardiac index, LV, RV volume/bsa larger for elite/pro runners, indicating cardiac remodeling (athlete's heart). Structurally, their heart has evolved to function better in endurance activity. **(Subject 2 (S2) in Table 4)**

Cardiac energetics is much more efficient for pro runners, they require less power to achieve a similar level of cardiac output compared to recreational runners.

**Approach for computing performance measure, proficiency score and personalized improvement guidance:** Following are there two goals -

1. Goal-1: Generate proficiency score to quantify the performance level (proficiency score) relative to elite, mid-level or amateur runner / athlete (FIG. 3C).
2. Goal-2: Generate personalized guidance / training plan for future runs, using the above data model, personalized cardiac digital twin and kinematics information (FIG. 3D).

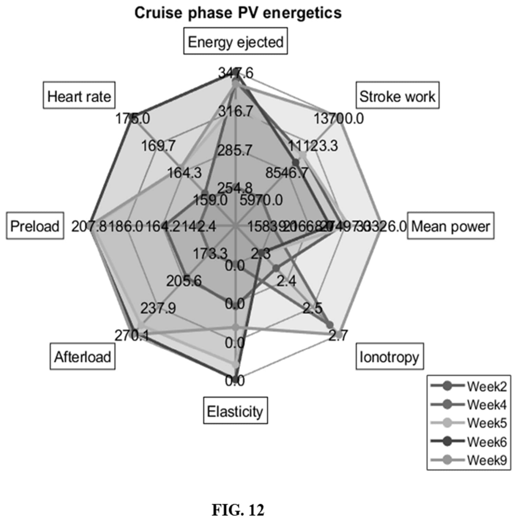

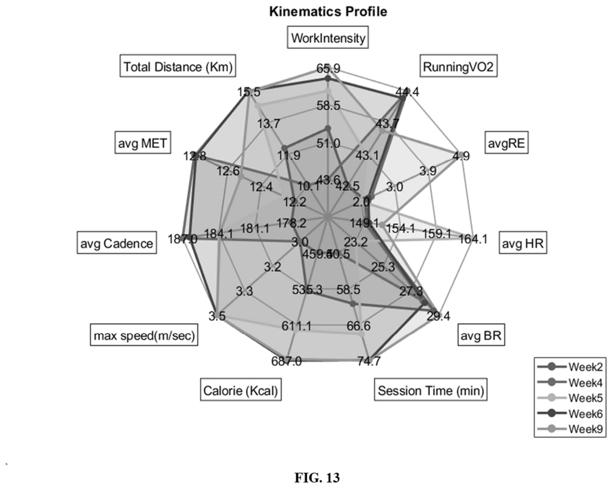

The performance measure is derived from the spider plot as depicted in FIG. 12 and FIG. 13. Each data point is the contour in the spider plot. There are two types of spider plots - (i) Cardiac Energetics (FIG. 12), (ii) Kinematics parameters (FIG. 13). For each subsegment (warm-up, ramp, cruise etc.) there would be separate spider plots for Cardiac Energetics. During the long-distance runs or long duration endurance activities there would be multiple spider plots, each for small time segments of few minutes. In the spider plots the values of the metrices increase as they go away from the center. For some metrics higher values are expected for elite athletes whereas for others less values are expected. This is linked with the polarity of the metrics. The contour would be used to compute the area within that contour with the polarity into consideration. The radial axis for certain metrics would be inverted to make the polarity of all metrics the same. From one individual athlete, multiple contours are obtained for a given endurance session. Multiple contours are generated by considering multiple time windows (e.g. 1-2 sec) in each time subsegment. These multiple contours are used to compute the distribution of the contour. The individual distribution of all metrices is taken for training the regression model (FIG. 3B).

Data from different proficiency levels (elite, mid-level, amateur) are considered for creating the model. Once the model is trained, during inference stage, the data from a new runner (test runner) can be used to derive the proficiency level (FIG. 3C).

Following are the broad steps to achieve the goals -

Create AI based models for elite/pro, mid-level and amateur runners - This is done by learning the distribution of various metrices for the three types of runners. For a new runner, find the distance (or probability) of the metrices from the above types of runners. This includes the metadata, running history, sensor data for recent runs.

Personalized training profile generation: Optimal profile selection could be based on the contour distribution information achieved from the AI aided regression model. A runner/coach might feel to increase power in one particular phase, say cruise, to achieve a certain running efficiency. These are the steps:

1. For a test run, the system 100 first computes different relevant metrics (kinematics) from the sensor data and starts the personalized CDT model.

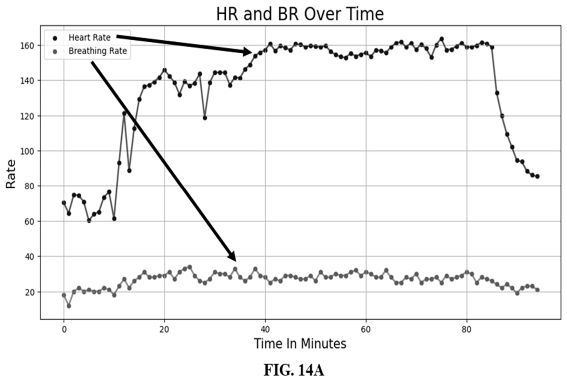

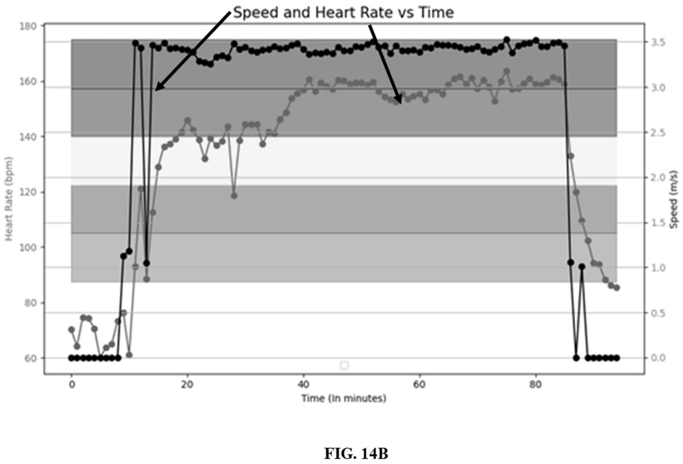

2. From the HR distribution plot and the comprehensive kinematic contour (FIG. 14A and 14B), automated phase segmentation are done to annotate start, ramp, dip, cruise, high and recovery segments.

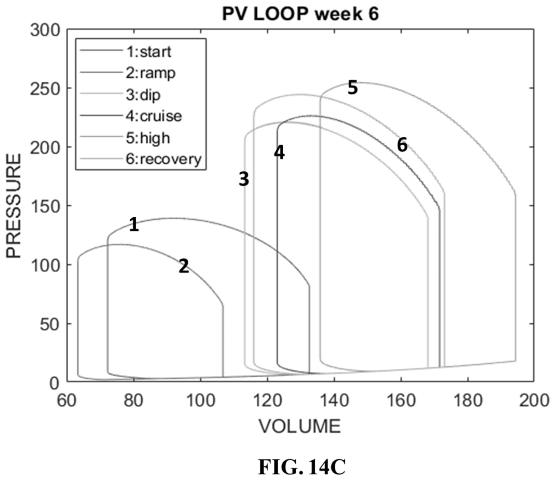

3. Cardio vascular model generates parameters and PV loops for each representative cycle for the annotated phases (FIG. 14C). Detailed dynamic analysis of these segmented phases are also done (FIG. 5A through FIG. 5F) to understand the dynamic variations.

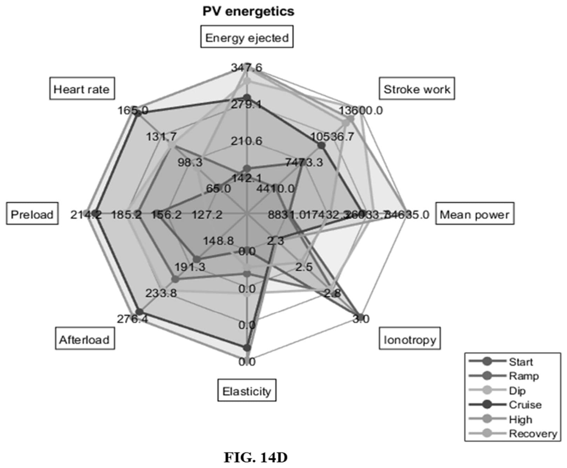

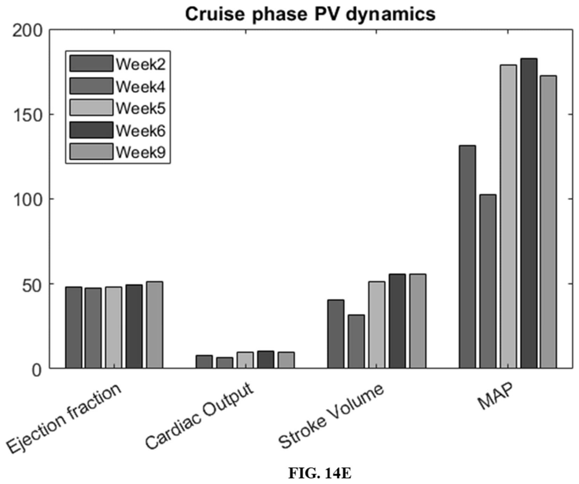

4. Contour energetics (FIG 12 and FIG. 14E) are derived from PV loop (FIG. 14C) using equations defined earlier. The energetics of 6 phases are depicted. Now if the target is to reduce cruise energetics (FIG. 14D), from the contour distribution profile of such cruise phases (FIG. 14E), the targeted profile could be chosen and to replicate that sample cruise phase energetics, suggestions could be (example ): "to run in tempo zone for 'X' amount of time followed by a dip for 'y' amount of time, maintaining HR at 'z', and then to speed up you may go to anaerobic threshold zone for 't' time."

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g. any kind of computer like a server or a personal computer, or the like, or any combination thereof. The device may also include means which could be e.g. hardware means like e.g. an application-specific integrated circuit (ASIC), a field-programmable gate array (FPGA), or a combination of hardware and software means, e.g. an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g. using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

1. A processor implemented method (200), the method comprising:

time synchronizing (202), via one or more hardware processors, sensor data acquired during an endurance activity performed by each subject among a plurality of subjects, wherein the sensor data represents a plurality of data types comprising electrocardiogram (ECG) data, accelerometer data providing speed, Gravity data, Global Positioning System (GPS) data and Barometer data acquired from a plurality of sensors worn by each subject, and wherein the plurality of subjects are a mix of a professional athlete, a mid-level athlete and an amateur athlete; segmenting (204), via the one or more hardware processors, each of the plurality of data types into a plurality of segments,

wherein a first step comprises segmenting by identification of i) an initial resting or warmup segment, ii) an Intense Activity (IA) segment and iii) a recovery segment post the IA segment based on Heart Rate (HR) variation and associated Metabolic Equivalent Task (MET); and wherein a second step comprises segmenting the IA segment into i) an initial ramp-up, ii) a cruise, iii) an occasional dip in speed, and iv) an occasional increase in speed and/or heartrate;

running (206), via the one or more hardware processors, a personalized Cardiac Digital Twin (CDT) model, built for each subject, on corresponding segments of each of the plurality data types to extract a plurality of sets of cardiopulmonary dynamics, wherein a plurality of sets of cardiopulmonary features are derived from the plurality of sets of cardiopulmonary dynamics for each subject, and wherein a distribution of the sets of cardiopulmonary features is processed via a feature transformation technique to obtain a transformed cardiopulmonary feature vector for each subject; extracting (208), via the one or more hardware processors, a set of kinematic features from one or more of the of the plurality of data types acquired during the endurance activities, wherein a distribution of the set of kinematic features is processed via a feature transformation technique to obtain a transformed kinematic feature vector for each subject; generating (210), via the one or more hardware processors, an annotated feature matrix comprising a plurality of features vectors representing the plurality of subjects via the transformed kinematic feature vector concatenated with the transformed cardiopulmonary feature vector, wherein each feature vector among a plurality of feature vectors of the annotated feature matrix is annotated with a proficiency score of each of the subject for the endurance activity; and creating (212), via the one or more hardware processors, a plurality of trained data regression models using the annotated feature matrix for predicting the proficiency score for the professional athlete, the mid-level athlete and the amateur athlete.

2. The processor implemented method as claimed in claim 1, wherein during inference personalized guidance and training plan for future runs of a test subject is generated based on the predicted proficiency score, a personalized CDT of the subject, and a set of kinematic and cardiopulmonary features extracted for the test subject.

3. The processor implemented method as claimed in claim 2, wherein the personalized guidance and training plan generation comprises determining a difference of the kinematic features and cardiopulmonary features for the test subject from the professional athlete and the mid-level athlete depending upon the predicted proficiency score of the test subject to identify a plurality of measures to be focused upon for improvement with reference the a mid-level athlete later progressing towards the professional athlete or an amateur athlete progressing towards mid-level.

4. The processor implemented method as claimed in claim 1, wherein the personalized CDT model is built using i) a plurality of cardiac structural parameters obtained from MRI and Echo test of each subject, ii) a plurality of subject-specific baseline clinical parameters, and iii) body physique and heart associated metadata of each subject.

5. The processor implemented method as claimed in claim 1, wherein the set of cardiopulmonary features comprise metabolic equivalent of task (MET), (av) arteriovenous, (per) perfusion, pva: pressure volume area, mep: mean power, VE: ventricular efficiency, Heart rate (HR), Energy ejected (EE), Stroke work (SW), mep: mean power, VE: ventricular efficiency, ESP end systolic pressure, EDV: end diastolic volume, ESPVR: end systolic pressure volume ratio, EDPVR: end diastolic pressure volume ratio, Mean power (Pmean), Cardiac output (CO), Stroke volume (SV), Ejection Fraction (EF), and Mean arterial pressure (MAP).

6. The processor implemented method as claimed in claim 1, wherein the set of kinematics features comprise Work Intensity (WI), Running VO2 (VO2run), Average running efficiency (REavg), REconomy, Average heart rate (HRavg), Average breathing rate (BRavg), Session time (ST), Calorie (Cal), maximum speed(Smax), Average Cadence(Cadavg), Average MET (METavg) , and Total distance (TD).

7. A system (100) comprising:

   a memory (102) storing instructions;
   one or more Input/Output (I/O) interfaces (106); and
   one or more hardware processors (104) coupled to the memory (102) via the one or more I/O interfaces (106), wherein the one or more hardware processors (104) are configured by the instructions to:

   time synchronize sensor data acquired during an endurance activity performed by each subject among a plurality of subjects, wherein the sensor data represents a plurality of data types comprising electrocardiogram (ECG) data, accelerometer data providing speed, Gravity data, Global Positioning System (GPS) data and Barometer data acquired from a plurality of sensors worn by each subject, and wherein the plurality of subjects are a mix of a professional athlete, a mid-level athlete and an amateur athlete;
   segment each of the plurality of data types into a plurality of segments,

   wherein a first step comprises segmenting by identification of i) an initial resting or warmup segment, ii) an Intense Activity (IA) segment and iii) a recovery segment post the IA segment based on Heart Rate (HR) variation and associated Metabolic Equivalent Task (MET); and
   wherein a second step comprises segmenting the IA segment into i) an initial ramp-up, ii) a cruise, iii) an occasional dip in speed, and iv) an occasional increase in speed and/or heartrate;

   run a personalized Cardiac Digital Twin (CDT) model, built for each subject, on corresponding segments of each of the plurality data types to extract a plurality of sets of cardiopulmonary dynamics, wherein a plurality of sets of cardiopulmonary features are derived from the plurality of sets of cardiopulmonary dynamics for each subject, and wherein a distribution of the sets of cardiopulmonary features is processed via a feature transformation technique to obtain a transformed cardiopulmonary feature vector for each subject;
   extract a set of kinematic features from one or more of the of the plurality of data types acquired during the endurance activities, wherein a distribution of the set of kinematic features is processed via a feature transformation technique to obtain a transformed kinematic feature vector for each subject;
   generate an annotated feature matrix comprising a plurality of features vectors representing the plurality of subjects via the transformed kinematic feature vector concatenated with the transformed cardiopulmonary feature vector, wherein each feature vector among a plurality of feature vectors of the annotated feature matrix is annotated with a proficiency score of each of the subject for the endurance activity; and
   create trained data regression models using the annotated feature matrix for predicting the proficiency score for the professional athlete, the mid-level athlete and the amateur athlete.

8. The system as claimed in claim 7, wherein during inference personalized guidance and training plan for future runs of a test subject is generated based on the predicted proficiency score, a personalized CDT of the subject, and a set of kinematic and cardiopulmonary features extracted for the test subject.

9. The system as claimed in claim 8, wherein the personalized guidance and training plan generation comprises determining a difference of the kinematic features and cardiopulmonary features for the test subject from the professional athlete and the mid-level athlete depending upon the predicted proficiency score of the test subject to identify a plurality of measures to be focused upon for improvement with reference the a mid-level athlete later progressing towards the professional athlete or an amateur athlete progressing towards mid-level.

10. The system as claimed in claim 7, wherein the personalized CDT model is built using i) a plurality of cardiac structural parameters obtained from MRI and Echo test of each subject, ii) a plurality of subject-specific baseline clinical parameters, and iii) body physique and heart associated metadata of each subject.

11. The system as claimed in claim 7, wherein the set of cardiopulmonary features comprise metabolic equivalent of task (MET), (av) arteriovenous, (per) perfusion, pva: pressure volume area, mep: mean power, VE: ventricular efficiency, Heart rate (HR), Energy ejected (EE), Stroke work (SW), mep: mean power, VE: ventricular efficiency, ESP end systolic pressure, EDV: end diastolic volume, ESPVR: end systolic pressure volume ratio, EDPVR: end diastolic pressure volume ratio, Mean power (Pmean), Cardiac output (CO), Stroke volume (SV), Ejection Fraction (EF), and Mean arterial pressure (MAP).

12. The system as claimed in claim 7, wherein the set of kinematics features comprise Work Intensity (WI), Running VO2 (VO2run), Average running efficiency (REavg), REconomy, Average heart rate (HRavg), Average breathing rate (BRavg), Session time (ST), Calorie (Cal), maximum speed (Smax), Average Cadence (Cadavg), Average MET (METavg), and Total distance (TD).

13. One or more non-transitory machine-readable information storage mediums comprising one or more instructions which when executed by one or more hardware processors cause:

> time synchronizing sensor data acquired during an endurance activity performed by each subject among a plurality of subjects, wherein the sensor data represents a plurality of data types comprising electrocardiogram (ECG) data, accelerometer data providing speed, Gravity data, Global Positioning System (GPS) data and Barometer data acquired from a plurality of sensors worn by each subject, and wherein the plurality of subjects are a mix of a professional athlete, a mid-level athlete and an amateur athlete;
> segmenting each of the plurality of data types into a plurality of segments,
>
>> wherein a first step comprises segmenting by identification of i) an initial resting or warmup segment, ii) an Intense Activity (IA) segment and iii) a recovery segment post the IA segment based on Heart Rate (HR) variation and associated Metabolic Equivalent Task (MET); and
>> wherein a second step comprises segmenting the IA segment into i) an initial ramp-up, ii) a cruise, iii) an occasional dip in speed, and iv) an occasional increase in speed and/or heartrate;
>
> running a personalized Cardiac Digital Twin (CDT) model, built for each subject, on corresponding segments of each of the plurality data types to extract a plurality of sets of cardiopulmonary dynamics, wherein a plurality of sets of cardiopulmonary features are derived from the plurality of sets of cardiopulmonary dynamics for each subject, and wherein a distribution of the sets of cardiopulmonary features is processed via a feature transformation technique to obtain a transformed cardiopulmonary feature vector for each subject;
> extracting a set of kinematic features from one or more of the of the plurality of data types acquired during the endurance activities, wherein a distribution of the set of kinematic features is processed via a feature transformation technique to obtain a transformed kinematic feature vector for each subject;
> generating an annotated feature matrix comprising a plurality of features vectors representing the plurality of subjects via the transformed kinematic feature vector concatenated with the transformed cardiopulmonary feature vector, wherein each feature vector among a plurality of feature vectors of the annotated feature matrix is annotated with a proficiency score of each of the subject for the endurance activity; and
> creating a plurality of trained data regression models using the annotated feature matrix for predicting the proficiency score for the professional athlete, the mid-level athlete and the amateur athlete.

14. The one or more non-transitory machine-readable information storage mediums of claim 13, wherein during inference personalized guidance and training plan for future runs of a test subject is generated based on the predicted proficiency score, a personalized CDT of the subject, and a set of kinematic and cardiopulmonary features extracted for the test subject.

15. The one or more non-transitory machine-readable information storage mediums of claim 14, wherein the personalized guidance and training plan generation comprises determining a difference of the kinematic features and cardiopulmonary features for the test subject from the professional athlete and the mid-level athlete depending upon the predicted proficiency score of the test subject to identify a plurality of measures to be focused upon for improvement with reference the a mid-level athlete later progressing towards the professional athlete or an amateur athlete progressing towards mid-level.

System**100**

Processor(s) **104**

I/O Interface(s) **106**

Memory **102**

Database **108**

Modules **110**

FIG. 1

200

time synchronizing sensor data acquired during an endurance activity performed by each subject among a plurality of subjects, wherein the sensor data represents a plurality of data types comprising ECG data, accelerometer data providing speed, Gravity data, GPS data and Barometer data acquired from a plurality of sensors worn by each subject, and wherein the plurality of subjects are a mix of a professional athlete, a mid-level athlete and an amateur athlete

202

segmenting each of the plurality of data types into a plurality of segments,

wherein a first step comprises segmenting by identification of i) an initial resting or warmup segment, ii) an Intense Activity (IA) segment and iii) a recovery segment post the IA segment based on Heart Rate (HR) variation and associated Metabolic Equivalent Task (MET); and

wherein a second step comprises segmenting the IA segment into i) an initial ramp-up, ii) a cruise, iii) an occasional dip in speed, and iv) an occasional increase in speed and/or heartrate

204

running a personalized Cardiac Digital Twin (CDT) model, built for each subject, on corresponding segments of each of the plurality data types to extract a plurality of sets of cardiopulmonary dynamics, wherein a plurality of sets of cardiopulmonary features are derived from the plurality of sets of cardiopulmonary dynamics for each subject, and wherein a distribution of the sets of cardiopulmonary features is processed via a feature transformation technique to obtain a transformed cardiopulmonary feature vector for each subject

206

A

**FIG. 2A**

200

A

extract a set of kinematic features from one or more of the of the plurality of data types acquired during the endurance activities, wherein a distribution of the set of kinematic features is processed via a feature transformation technique to obtain a transformed kinematic feature vector for each subject — 208

generating an annotated feature matrix comprising a plurality of features vectors representing the plurality of subjects via the transformed kinematic feature vector concatenated with the transformed cardiopulmonary feature vector, wherein each feature vector among a plurality of feature vectors of the annotated feature matrix is annotated with a proficiency score of each of the subject for the endurance activity — 210

creating trained data regression models using the annotated feature matrix for predicting the proficiency score for the professional athlete, the mid-level athlete and the amateur athlete — 212

**FIG. 2B**

FIG. 3A

FIG. 3B

FIG. 3C

**FIG. 3D**

Speed, Heart Rate, and Relative Altitude Over Time

FIG. 4

FIG. 5A

FIG. 5B

Segment 3: dip

FIG. 5C

FIG. 5D

**FIG. 5E**

FIG. 5F

**FIG. 6A**

FIG. 6B

**FIG. 6C**

FIG. 7

**Kinematics Profile Comparison**

**FIG. 8**

**PV LOOP**

FIG. 9

**FIG. 10**

**FIG. 11**

FIG. 12

**FIG. 13**

FIG. 14A

**FIG. 14B**

FIG. 14C

**FIG. 14D**

**FIG. 14E**

**EUROPEAN SEARCH REPORT**

Europäisches
Patentamt
European
Patent Office
Office européen
des brevets

**Application Number**

EP 25 21 0011

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | JAKIM BERNDSEN ET AL: "Pace my race", RECOMMENDER SYSTEMS, ACM, 2 PENN PLAZA, SUITE 701NEW YORKNY10121-0701USA, 10 September 2019 (2019-09-10), pages 246-250, XP058440608, DOI: 10.1145/3298689.3346991 ISBN: 978-1-4503-6243-6 * abstract * * section 1 second paragraph * * section 2.1, second paragraph * * section 3.2 first sentence * * section 3.4 second paragraph * ----- | 1-15 | INV.<br>G16H20/30<br>A61B5/00<br>G16H50/20<br><br>ADD.<br>A61B5/024<br>A61B5/08<br>A61B5/11<br>G16H50/30 |
| A | US 2024/070854 A1 (EL-SALLAM AMAR [AU] ET AL) 29 February 2024 (2024-02-29) * abstract * * paragraphs [0004], [0028] - [0035], [0113]; claim 1 * ----- | 1-15 | |
| A | US 9 826 908 B2 (SMARTHEALTH ELECTRONICS LTD [CN]) 28 November 2017 (2017-11-28) * abstract * * paragraphs [0005], [0027], [0038] - [0040], [0061], [0072], [0079], [0208], [0209], [0220], [0257]; claim 1 * ----- | 1-15 | **TECHNICAL FIELDS SEARCHED (IPC)**<br><br>G16H<br>A61B |
| A | US 2017/266499 A1 (SANDERS DANIEL [US] ET AL) 21 September 2017 (2017-09-21) * abstract * * column 3, lines 50-51 * * column 5, line 59 - column 6, line 4 * * column 10, lines 13-16 * ----- -/-- | 1-15 | |

The present search report has been drawn up for all claims

| Place of search | Date of completion of the search | Examiner |
|---|---|---|
| Munich | 12 March 2026 | Ricciardi, Maurizio |

EPO FORM 1503 03.82 (P04C01)

page 1 of 2

48

Europäisches
Patentamt

European
Patent Office

Office européen
des brevets

# EUROPEAN SEARCH REPORT

**Application Number**

EP 25 21 0011

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2023/414143 A1 (SEPPÄNEN MIKKO [FI] ET AL) 28 December 2023 (2023-12-28) <br> * abstract * <br> * paragraphs [0002], [0006], [0037], [0064], [0101], [0163], [0192], [0253] * <br><br> ----- | 1-15 | |
| | | | **TECHNICAL FIELDS SEARCHED (IPC)** |

The present search report has been drawn up for all claims

| Place of search | Date of completion of the search | Examiner |
|---|---|---|
| Munich | 12 March 2026 | Ricciardi, Maurizio |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

    .....................................................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 25 21 0011

This annex lists the patent family members relating to the patent documents cited in the above-mentioned European search report.
The members are as contained in the European Patent Office EDP file on
The European Patent Office is in no way liable for these particulars which are merely given for the purpose of information.

12-03-2026

| Patent document cited in search report | Publication date | Patent family member(s) | Publication date |
|---|---|---|---|
| US 2024070854 A1 | 29-02-2024 | AU 2022204095 A1 | 01-12-2022 |
| | | US 2024070854 A1 | 29-02-2024 |
| US 9826908 B2 | 28-11-2017 | CN 104840191 A | 19-08-2015 |
| | | CN 105852846 A | 17-08-2016 |
| | | EP 3087917 A1 | 02-11-2016 |
| | | US 2016317044 A1 | 03-11-2016 |
| US 2017266499 A1 | 21-09-2017 | CN 109155153 A | 04-01-2019 |
| | | CN 109155154 A | 04-01-2019 |
| | | EP 3430546 A1 | 23-01-2019 |
| | | EP 3430547 A1 | 23-01-2019 |
| | | US 2017266498 A1 | 21-09-2017 |
| | | US 2017266499 A1 | 21-09-2017 |
| | | US 2017266500 A1 | 21-09-2017 |
| | | US 2017266501 A1 | 21-09-2017 |
| | | US 2022044806 A1 | 10-02-2022 |
| | | US 2022044807 A1 | 10-02-2022 |
| | | US 2023298748 A1 | 21-09-2023 |
| | | US 2023307124 A1 | 28-09-2023 |
| | | US 20260018283 A1 | 15-01-2026 |
| | | WO 2017161013 A1 | 21-09-2017 |
| | | WO 2017161021 A1 | 21-09-2017 |
| | | WO 2017161025 A1 | 21-09-2017 |
| | | WO 2017161029 A1 | 21-09-2017 |
| US 2023414143 A1 | 28-12-2023 | US 2020261011 A1 | 20-08-2020 |
| | | US 2023414143 A1 | 28-12-2023 |

EPO FORM P0459

For more details about this annex : see Official Journal of the European Patent Office, No. 12/82

**REFERENCES CITED IN THE DESCRIPTION**

**Patent documents cited in the description**

- IN 202421082908
- IN 201921029536
- IN 202121010972
- IN 202321080595
- IN 202221033450
- IN 368504
- IN 439754
- IN 545836